# EUROPEAN PATENT APPLICATION

(11) **EP 2 153 732 A1**
(43) Date of publication of application: **17.02.2010**
(21) Application number: 08161734.2
(22) Date of filing: 04.08.2008
(51) Int. Cl.: A23K 1/00, A23L 1/00, A23L 1/212, A23L 1/30, A61K 9/16, A61K 9/20, A61K 9/50, A61K 36/31

(54) **Production of Beadlets Comprising Hygroscopic Plant Extracts**

(71) Applicant: DSM IP Assets B.V., 6411 TE Heerlen (NL)
(72) Inventor: The designation of the inventor has not yet been filed
(74) Representative: Kurt, Manfred

(57) **Abstract**

The present invention relates a process of production of beadlets comprising hygroscopic plant extracts in a matrix comprising at least one starch and/or starch derivative, to such beadlets and to the use of such specific beadlets in food (for humans and animals) as well as in premixes.

## Description

The present invention relates a process of production of beadlets comprising hygroscopic plant extracts in a matrix comprising at least one starch and/or starch derivative, to such beadlets and to the use of such specific beadlets.

Hygroscopic plant extract in the context of the present invention relates to plant extracts, which comprises amorphous sugar(s) or other hygroscopic compounds.

Plant extracts can be made by methods known in the arts including a polar extract such as an aqueous extract, an alcohol extract (e.g., ethanol, methanol, hexane, hydroalcohol,) or a non-polar extract (e.g., isooctane).

Preferred hygroscopic plant extracts in the context of the present invention are extracts from cruciferous plants.

Plant extracts of cruciferous plants such as rocket, broccoli, cabbage, watercress, radish cabbage, bok choy, collards, brussels sprouts, kohlrabi, kale, mustard greens, turnip greens, and cauliflower are known having a good effect on various aspects of health. These vegetables contain phytochemicals that help to strengthen the immune system and thereby help the body to build resistance against viruses and diseases. Cruciferous vegetables are rich in the antioxidant vitamins C, E, and beta-carotene, and are a good source of dietary fibre.

Cruciferous vegetables also contain glucoraphanin and other isothiocyanates, which are believed to stimulate the production of protective enzymes in the body. These powerful enzymes detoxify toxins and other problem-causing agents and flush them from the body.

The term *"cruciferous plant extract"* is defined as an extract made from a cruciferous plant. The extract can be obtained from any part of the plant, usually from the seeds or sprouts.

The extracts of these plants allow a wide range of uses. The extract can be incorporated in many forms of application. Food products and dietary supplements is one of these fields. It is possible to have the positive effects of the ingredients without the need of eating the whole plant or even without having the smell or taste of the plants.

Plant extracts are often hygroscopic. They tend to take up water from humid air and become sticky or even liquefy at ambient conditions. This makes these extracts very difficult to dry, process and handle.

A very typical form of formulation of substances used in consumer products (such as for example food products) are powders. Powders can be produced by spray drying or freeze drying processes. These formulations can contain a high content of one water soluble and/or water-dispersible hygroscopic substance as well as mixture of at least two water soluble and/or water-dispersible hygroscopic substances. But when using powders comprising hygroscopic substances, there are a few problems:
- The powders are still hygroscopic.
- Dry powders are usually dusty and therefore the handling can be difficult.

To solve the problem of hygroscopicity, additives like maltodextrins, silica or Ca-stearate are added to the extract. But in many cases, the hygroscopicity of the resulting powders is still too high for the use in applications like tabletting.

Another well known form of preparations are beadlets. Beadlets provide superior handling properties in that they are not dusty and possess good flowablity characteristics. Beadlets are solely known for fat-soluble substances.

Beadlets (comprising fat-soluble substances) and their methods of productions are known from the prior art. These beadlets comprise fat-soluble (lipophilic, hydrophobic) substances. Such beadlets and their process for production are for example known from US2006/0115534 and US4670247. These beadlets usually have good storage stability, but the concentration of the fat-soluble substances in such beadlets is low. Usually the content is between 5 - 15 weight-% (wt-%), based on the total weight of the beadlet.

Another disadvantage is that the production of such beadlets requires an emulsification or dispersion step to distribute the water-insoluble active in the aqueous matrix phase. Therefore, the matrix material needs to have emulsifying properties or an additional emulsifier is required.

The goal of the present invention was to find a process for producing formulations comprising water-soluble or water-dispersible hygroscopic substances, which
- allows producing non-hygroscopic formulations with a high amount of water-soluble or water-dispersible hygroscopic substances
- produces storage stable formulations
- is a simple process.

Surprisingly, it has been found that the use of the powder catch process allows producing such formulations, which are in the form of beadlets having the above mentioned advantages. The beadlets comprise a hygroscopic plant extract (or a mixture of various different plant extracts) and one starch and/or starch derivative (or a mixture of starches and/or starch derivatives) as a matrix material. Furthermore these beadlets are coated with a layer of the powder catch medium.

Therefore, the present invention relates to a process for preparing beadlets, which comprise at least one hygroscopic plant extract, comprising:
(a) forming an aqueous solution of
   (i) at least one hygroscopic plant extract and
   (ii) at least one starch and/or at least one starch derivative,
(b) converting the solution into a dry powder by spray drying into a collecting powder.

Such process are known from the prior art. It can be found for example in US6444227 or W004062382. These references are hereby incorporated by reference.

Preferred hygroscopic plant extracts are cruciferous plant extracts. Plant extracts of cruciferous plants are for example rocket, broccoli, cabbage, watercress, radish cabbage, bok choy, collards, brussels sprouts, kohlrabi, kale, mustard greens, turnip greens and cauliflower.

Therefore a preferred embodiment of the present invention relates to a process for preparing beadlets, which comprise at least one cruciferous plant extract.

A more preferred embodiment of the present invention relates to a process for preparing beadlets, which comprise at least one hygroscopic plant extract, which is a cruciferous plant extract chosen from the group consisting of rocket, broccoli, cabbage, watercress, radish cabbage, bok choy, collards, brussels sprouts, kohlrabi, kale, mustard greens, turnip greens and cauliflower, preferably broccoli.

The hygroscopic plant extracts (preferably cruciferous plant extracts) are formulated in a beadlet by a matrix material, which comprises at least one starch and/or starch derivative.

Starch having the chemical formula (C₆H₁₀O₅)ₙ is a polysaccharide carbohydrate consisting of a large number of glucose monosaccharide units joined together by glycosidic bonds.

All plant seeds and tubers contain starch. Starches are commonly extracted from plants, such as corn, sorghum, wheat, rice, tapioca, arrowroot, sago, potato, quinoa and amaranth.

Natural starches contain usually amylase and amylopectin molecules. The content of amylase in natural starches can vary from 0 wt-% (for example waxy corn starch and waxy rice starch) up to about 85wt-% (High amylase corn starch). Normal starches contain about 25 wt-% of amylase. As a consequence thereof the content of amylopectin is between 15 and 100 wt-%.

It is also possible to use starch derivatives (modified starches) including hydrolysed starches. The starches can be modified in various manners. It can be done physically and chemically.

Pregelatinised starches are examples of physically modified starches.

Acidic modified, oxidized, cross-linked, starch esters, starch ethers and cationic starches are examples of chemically modified starches.

Dextrins like maltodextrin or yellow dextrin are examples starch derivatives obtained by partial hydrolysis.

In a preferred process according to the present invention the starches or starch derivatives are chosen from the group consisting of amylopectin and pregelatinised starches.

The matrix of the beadlets of the present invention can also comprise additional compounds, such as sugar (sucrose).

A preferred process according to the present invention is a powder catch process. Such a process is known from the prior art (for example from W004062382). As a result of such a powder catch process the beadlets are covered by a layer of the powder.

Therefore, the beadlets produced according to this process are preferably covered by a layer of the powder catch medium. This layer (coating) is in the form of a powder coating.

The powder catch medium is a compound (or a mixture of compounds), which is able to absorb moisture and to form a powder coating. Suitable powder catch media are i.e. starches, silicate or phosphate compounds. Preferred powder catch media are starches (such as i.e. corn starch), calcium silicate, calcium aluminium silicate and tri-calcium phosphate. Most preferred are starches, especially corn starch.

Beadlets are a well known form of formulation for fat-soluble substances. An important advantage of the generally spherical beadlets is that they are not dusty and that they posses excellent free flowing characteristics, which are very desirable for manufacturing and formulating operations.

Usually the size of a beadlet is from 50 □m to 1,000 □m (preferably from 250□m to 850 □m). The sizes can be smaller or larger. The size of a beadlet can be determined according to well known methods, such as (scanning) electron microscopy.

A suitable method to produce beadlets as disclosed and described above is for example described in WO 2004/062382. This reference is hereby incorporated.

The process according to the present invention surprisingly allows producing beadlets with a high amount of hygroscopic plant extract (preferably cruciferous plant extract) with good overall properties.

The process as described in the present patent application can be used not only to produce beadlets with a high amount of hygroscopic plant extract (preferably cruciferous plant extract), but also with a low amount thereof. The amount can be as low as 1 wt-%, based on the total weight of the beadlets, Usually the content of the hygroscopic plant extract (preferably cruciferous plant extract) in the beadlets is at least 5 wt-%, preferably at least 10 wt-%, based on the total weight of the beadlets.

A preferred process according to present invention relates to a process according as described above wherein the beadlets comprise at least 30 wt-%, based on the total weight of the beadlets, of at least one hygroscopic plant extract (preferably cruciferous plant extract).

A further preferred process according to present invention relates to a process according as described above wherein the beadlets comprise up to 85 wt-%, based on the total weight of the beadlets, of at least one hygroscopic plant extract (preferably cruciferous plant extract).

A preferred process according to present invention relates to a process wherein the beadlets comprise up to 80 wt-%, based on the total weight of the beadlets, of at least one hygroscopic plant extract (preferably cruciferous plant extract).

A preferred process according to present invention relates to a process wherein the beadlets comprise at least 5 wt-%, based on the total weight of the beadlets, more preferred 20 wt-%, of at least one starch and/or at least one starch derivative (matrix material) and of the powder coating layer.

A preferred process according to present invention relates to a process wherein the beadlets comprise
at least 5 wt-%, based on the total weight of the beadlets, of powder coating layer.

A more preferred process according to present invention relates to a process wherein the beadlets comprise
(i) 5 wt-% - 85 wt-%, preferably 30 wt-% - 80 wt-%, more preferably 40 wt-% - 70 wt-%, based on the total weight of the beadlets, of at least one hygroscopic plant extract (preferably cruciferous plant extract), and
(ii) 5 wt-% - 90 wt-%, preferably 25 wt-% - 50 wt-%, based on the total weight of the beadlets, of at least one starch and/or at least one starch derivative, and
(iii) 5 wt-% - 50 wt-%, preferably 5 wt-% - 20 wt-%, based on the total weight of the beadlets, of powder coating.

As mentioned above the matrix of the beadlets of the present invention can also comprise additional compounds. A preferred compound is sugar (sucrose).

Beadlets comprising a high amount (at least 30 wt-%) of hygroscopic plant extract (preferably cruciferous plant extract)in a matrix comprising at least one starch and/or at least one starch derivative are not known from the prior art.

A further embodiment of the present invention relates to beadlets (B1) comprising
(i) at least 30 wt-%, based on the total weight of the beadlets, of at least one hygroscopic plant extract (preferably cruciferous plant extract) and
(ii) at least one starch and/or at least one starch derivative.

The invention also relates to beadlets (B2) comprising
(i) up to 85 wt-%, based on the total weight of the beadlets, of at least one hygroscopic plant extract (preferably cruciferous plant extract), and
(ii) at least one starch and/or at least one starch derivative.

Preferred beadlets (B1') according to present invention comprise
(i) at least 30 wt-%, based on the total weight of the beadlets, of at least one hygroscopic plant extract (preferably cruciferous plant extract) and
(ii) up to 70 wt-%, based on the total weight of the beadlets, of at least one starch and/or at least one starch derivative.

The beadlets (B2') comprise
(i) up to 85 wt-%, based on the total weight of the beadlets, of at least one hygroscopic plant extract (preferably cruciferous plant extract), and
(ii) at least 15 wt-%, based on the total weight of the beadlets, of at least one starch and/or at least one starch derivative.

Preferred starch covered beadlets according to present invention comprise at least 5 wt-%, based on the total weight of the beadlets, of the power coating layer.
Therefore (B1), (B1'), (B2) and (B2') preferably comprise 5 wt-%, based on the total weight of the beadlets, of powder coating layer.

More preferred beadlets according to the present invention (B3) comprise
(i) 30 wt-% - 80 wt-%, preferably 40 wt-% - 70 wt-%, based on the total weight of the beadlets, of at least one cruciferous plant extract chosen from the group consisting of rocket, broccoli, cabbage, watercress, radish cabbage, bok choy, collards, brussels sprouts, kohlrabi, kale, mustard greens, turnip greens and cauliflower, and
(ii) 15 wt-% - 65 wt-%, preferably 25 wt-% - 50 wt-%, based on the total weight of the beadlets, of at least one starch and/or starch derivative chosen from the groups consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches (preferred are starches with a high amount of amylopectin and pregelatinised starches), and
(iii) 5 wt-% - 50 wt-%, preferably 5 wt-% - 20 wt-%, based on the total weight of the beadlets, of powder coating chosen from the group consisting of starches (preferably corn starch), silicate and phosphate compounds.

In more preferred process according to the present invention the starches or starch derivatives are chosen from the group consisting of amylopectin and pregelatinised starches.

Especially preferred beadlets according to the present invention (B3') comprise
(i) 30 wt-% - 80 wt-%, based on the total weight of the beadlets, of at least one broccoli extract, and
(ii) 15 wt-% - 65 wt-%, based on the total weight of the beadlets, of at least one starch and/or starch derivative chosen from the groups consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches (preferred are starches with a high amount of amylopectin and pregelatinised starches), and
(iii) 5 wt-% - 50 wt-%, based on the total weight of the beadlets, of powder coating chosen from the group consisting of starches (preferably corn starch), silicate and phosphate compounds.

Most preferred beadlets according to the present invention (B3") comprise
(i) 40 wt-% - 70 wt-%, based on the total weight of the beadlets, of at least one broccoli extract, and
(ii) 25 wt-% - 50 wt-%, based on the total weight of the beadlets, of at least one starch and/or starch derivative chosen from the groups consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches (preferred are starches with a high amount of amylopectin and pregelatinised starches), and
(iii) 5 wt-% - 20 wt-%, based on the total weight of the beadlets, of powder coating chosen from the group consisting of starches (preferably corn starch), silicate and phosphate compounds.

Further more preferred beadlets are beadlets (B1), (B1'), (B2), (B2'), (B2"), (B3), (B3') and (B3") additionally comprising sugar (sucrose). These beadlets (B4) comprise 5 - 25 wt-%, preferably 10 - 20 wt-%, based on the total weight of the beadlets, of sucrose.

A further embodiment according to the present invention relates to the use of the beadlets (B1), (B1'), (B2), (B2'), (B3), (B3'), (B3") and (B4) in food products (for humans and/or animals), dietary supplements as well in the production of food products and dietary supplements.

Depending what kind of cruciferous plant extract and/or matrix material are used, the food product is suitable for humans or animals. In some cases a food product could be consumed by humans and animals.

Food products (for humans and/or animals) in the context of the present comprise liquid and solid food products as well as paste-like and or gel like. The food products comprise food for humans as well as for animals (especially ruminants, poultry and swine).

A dietary supplement, also known as food supplement or nutritional supplement, is a preparation intended to supply nutrients, such as vitamins, minerals, fatty acids or amino acids that are missing or are not consumed in sufficient quantity in a person's diet.

The food product can be in a ready-to-consume form that means a form, which is suitable to eat without further proceedings. But it is also possible that food product is a form, which needs further proceedings, like heating, dissolving, diluting, etc.

Suitable human food products can be drinks, soups, bars (cereal, chocolate), dairy products, etc.

Suitable animal food products (feed products) can be in any commonly used form.

Therefore a further embodiment of the present invention relates to human and animal food products and to human and animal dietary supplements comprising beadlets as described above.

Premixes are a convenient usage form for the food producers but are a critical medium for various ingredients due to pH, ionic strength and water activity values, which can negatively affect viability of various ingredients. But the beadlets according to the present invention eliminate (or at least strongly minimize) such problems.

The beadlets according to the present invention can also be used in premixes for food products (for humans and/or animals) and for dietary supplements (for humans and/or animals

A further embodiment of the present invention is a premix for food products (for humans and/or animals) and for dietary supplements (for humans and/or animals) comprising beadlets according to the present invention.

Functional ingredients like vitamins and trace elements are often added to food or feed products as well as to premixes.

The following examples serve to illustrate the invention. The percentages are expressed in weight percentages and the temperatures are degrees Celsius, if not otherwise defined.

### Example 1: Formulation of broccoli extract in a matrix comprising amylopectin

6 g of amylopectin were added to 20 ml of water. The mixture was heated and stirred until dissolution occurred. To this solution, 30g broccoli extract, dissolved in 40 g water were added under stirring. About 82 g of the solution was sprayed in a spraying pan in a bed of fluidized starch at about 5°C by means of a rotating spraying nozzle. The so-obtained beadlets were separated from excess starch by sieving and dried. There were obtained ca. 83 g of dry powder

## Claims

1. A process for preparing beadlets, which comprise at least one hygroscopic plant extract, comprising:
(a) forming an aqueous solution of
(i) at least one hygroscopic plant extract and
(ii) at least one starch and/or starch derivate,
(b) converting the solution into a dry powder by spray drying into a collecting powder.

2. Process according to claim 1, wherein the hygroscopic plant extract is a cruciferous plant extract.

3. Process according to claim 2 wherein the cruciferous plant extract is chosen from the group consisting of rocket, broccoli, cabbage, watercress, radish cabbage, bok choy, collards, brussels sprouts, kohlrabi, kale, mustard greens, turnip greens and cauliflower.

4. Process according to claim 2 or 3 wherein the cruciferous plant extract is a broccoli extract.

5. Process according to any of the preceding claims, wherein the starch and/or starch derivates are chosen from the group consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches.

6. Process according claim 1, 2, 3 or 4, wherein the starch and/or starch derivates are chosen from the group consisting of dextrins, amylopectin and pregelatinised starches.

7. Process according to any of the preceding claims, wherein the beadlets are covered by a powder coating.

8. Process according to any of the preceding claims, wherein the beadlets comprise at least 30 wt-%, based on the total weight of the beadlets, of at least one hygroscopic plant extract.

9. A process according to any of the preceding claims, wherein the beadlets comprise up to 85 wt-%, based on the total weight of the beadlets, of at least one hygroscopic plant extract.

10. A process according to any of claims 1 - 8, wherein the beadlets comprise up to 80 wt-%, based on the total weight of the beadlets, of at least one hygroscopic plant extract.

11. A process according to any of the preceding claims, wherein the beadlets comprise at least 5 wt-%, preferably 20 wt-%, based on the total weight of the beadlets, of at least one gum compound and of the powder coating layer.

12. A process according to any of the preceding claims, wherein the beadlets comprise at least 5 wt-%, based on the total weight of the beadlets, of powder coating layer.

13. Beadlets comprising
(i) at least 30 wt-%, based on the total weight of the beadlets, of at least one hygroscopic plant extract and
(ii) at least one starch and/or starch derivative.

14. Beadlets according to claim 13 comprising
(i) up to 85 wt-%, based on the total weight of the beadlets, of at least hygroscopic plant extract.

15. Beadlets according to claims 13 and 14 comprising
(i) at least 30 wt-%, based on the total weight of the beadlets, of at least one hygroscopic plant extract and
(ii) up to 70 wt-%, based on the total weight of the beadlets, of at least one starch and/or starch derivative.

16. Beadlets according to any of claim 13 - 15, wherein the hygroscopic plant extract is a cruciferous plant extract.

17. Beadlets according to any of claims 13 - 16 comprising
(i) 30 wt-% - 80 wt-%, preferably 40 wt-% - 70 wt-%, based on the total weight of the beadlets, of at least one cruciferous plant extract chosen from the group consisting of rocket, broccoli, cabbage, watercress, radish cabbage, bok choy, collards, brussels sprouts, kohlrabi, kale, mustard greens, turnip greens and cauliflower, and
(ii) 15 wt-% - 65 wt-%, preferably 25 wt-% - 50 wt-%, based on the total weight of the beadlets, of at least starch and/or starch derivates are chosen from the group consisting of corn starch, sorghum starch, wheat starch, rice starch, tapioca starch, arrowroot starch, sago starch, potato starch, quinoa starch and amaranth starch, pregelatinised starches, acidic modified starches, oxidized starches, cross-linked starches, starch esters, starch ethers, dextrins and cationic starches., and
(iii) 5 wt-% - 50 wt-%, preferably 5 wt-% - 20 wt-%, based on the total weight of the beadlets, of powder coating chosen from the group consisting of starches (preferably corn starch), silicate and phosphate compounds.

18. Beadlets according to claim 17, wherein the cruciferous plant extract is a broccoli extract.

19. Use of the beadlets according to any of claims 13 - 18 in food products (for humans and/or animals) and dietary supplements (for humans and/or animals).

20. Use of the beadlets according to any of claims 13 - 18 in premixes for food products (for humans and/or animals) and for dietary supplements (for humans and/or animals).

21. Food products (for humans and/or animals) and dietary supplements (for humans and/or animals) comprising beadlets according to any of claims 13 - 18.

22. Premix for food products (for humans and/or animals) and for dietary supplements (for humans and/or animals) comprising beadlets according to any of claims 13 - 18.
